(19) Europäisches Patentamt
European Patent Office
Office européen des brevets

(11) EP 4 018 922 B1

(12) **FASCICULE DE BREVET EUROPEEN**

(45) Date de publication et mention
de la délivrance du brevet:
**19.02.2025 Bulletin 2025/08**

(21) Numéro de dépôt: **21217684.6**

(22) Date de dépôt: **24.12.2021**

(51) Classification Internationale des Brevets (IPC):
***A61B 5/024*** *(2006.01)*

(52) Classification Coopérative des Brevets (CPC):
**A61B 5/02416**

(54) **PROCÉDÉ D ESTIMATION D'UN RYTHME CARDIAQUE OU D'UN RYTHME RESPIRATOIRE**

VERFAHREN ZUR SCHÄTZUNG EINES HERZ- ODER ATEMRHYTHMUS

METHOD FOR ESTIMATING A HEART RATE OR A RESPIRATORY RATE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **26.12.2020 FR 2014157**

(43) Date de publication de la demande:
**29.06.2022 Bulletin 2022/26**

(73) Titulaire: **Commissariat à l'Energie Atomique et
aux Energies
Alternatives
75015 Paris (FR)**

(72) Inventeur: **BONNET, Stéphane
38054 Grenoble cedex 09 (FR)**

(74) Mandataire: **INNOV-GROUP
209 Avenue Berthelot
69007 Lyon (FR)**

(56) Documents cités:
**US-A1- 2008 167 564    US-A1- 2015 105 666
US-A1- 2015 150 515    US-A1- 2017 042 432
US-A1- 2018 184 983**

## Description

### DOMAINE TECHNIQUE

Le domaine technique de l'invention est une estimation d'un rythme cardiaque ou d'un rythme respiratoire.

### ART ANTERIEUR

La photoplethysmographie (PPG) est une méthode optique non invasive qui permet d'évaluer les variations du volume sanguin dans les tissus superficiels par la variation de l'absorption de lumière dans ces tissus. Cette méthode permet l'estimation de paramètres physiologiques, comme le rythme cardiaque ou le taux d'oxygénation. Elle repose sur une mesure des variations de la lumière transmise ou rétrodiffusée par les tissus à l'aide d'un capteur optique.

Un exemple de mise en œuvre de la PPG est l'oxymétrie de pouls, réalisée à l'aide d'une pince enserrant un doigt ou le lobe d'une oreille. Ce type de dispositif comporte une source de lumière et un photodétecteur, le doigt ou le lobe étant disposé entre la source de lumière et le photodétecteur, selon une configuration de transmission. Dans d'autres applications de la PPG, la source de lumière et le photodétecteur sont situés côte à côte. C'est par exemple le cas des dispositifs de PPG intégrés dans des montres. Le photodétecteur détecte des photons rétrodiffusés par les tissus illuminés par la source de lumière. On parle de configuration en rétrodiffusion.

Quelle que soit la configuration, le signal détecté par le photodétecteur comporte une composante continue, à laquelle s'ajoute une composante pulsatile, cette dernière variant en fonction du rythme cardiaque. L'information relative au rythme cardiaque est contenue dans la composante pulsatile. Cette dernière peut être extraite par filtrage passe haut du signal détecté par le photodétecteur.

A partir de la composante pulsatile, un seuillage en amplitude permet d'identifier des instants caractéristiques, typiquement des extrema (maxima ou minima). L'intervalle temporel séparant les instants caractéristiques permet une estimation d'un rythme cardiaque. Un exemple d'application est par exemple donné dans US9778111B2.

Cependant, la composante pulsatile d'un signal optique de type PPG peut comporter des fluctuations d'intensité importantes. L'application des seuils d'intensité pour sélectionner les instants caractéristiques peut manquer de précision.

Le document US2015/0155015 décrit différentes méthodes pour estimer un rythme respiratoire d'une personne à partir de signaux représentatifs de l'activité cardiaque. Ce document décrit un algorithme de traitement élaboré appliqué à des signaux acquis selon une modalité ECG (Electrocardiogramme). En effet, chaque période d'un signal de type ECG comporte plusieurs pics.

Le traitement décrit dans US2015/0155015 vise à détecter le pic d'amplitude maximale dans chaque période. US2015/01555015 décrit également le traitement de signaux acquis selon une modalité optique, de type PPG, en considérant que l'activité cardiaque peut être estimée par une simple détection de maximas ou de minimas.

L'inventeur propose une méthode simple à mettre en œuvre, sans seuillage d'intensité, pour déterminer le rythme cardiaque, ou le rythme respiratoire, ou autre paramètre physiologique périodique à partir de mesures non invasives.

### EXPOSE DE L'INVENTION

Un premier objet de l'invention est un procédé de détermination d'une fréquence ou d'une période d'une évolution temporelle d'un paramètre physiologique, le paramètre physiologique variant de façon périodique en fonction du temps, sous l'effet d'une activité cardiaque ou respiratoire d'un utilisateur, le procédé comportant les étapes suivantes :

a) détection, par un détecteur, d'un signal représentatif du paramètre physiologique de l'utilisateur en différents instants ;
b) à partir des signaux détectés, obtention d'une fonction de mesure ;
c) obtention d'une composante pulsatile de la fonction de mesure, la composante pulsatile étant une fonction périodique dont la période dépend de l'activité cardiaque ou de l'activité respiratoire ;
d) à partir de la composante pulsatile, détermination du rythme cardiaque ou du rythme respiratoire ;

le procédé étant caractérisé en ce que l'étape d) comporte :

- di) obtention d'une expression fréquentielle de la composante pulsatile ;
- dii) sélection d'une fréquence dominante de l'expression fréquentielle ;
- diii) filtrage de l'expression fréquentielle à la fréquence dominante, de façon à obtenir une expression fréquentielle filtrée ;
- div) à partir de l'expression fréquentielle filtrée, obtention d'une fonction temporelle sinusoïdale à la fréquence dominante ;
- dv) prise en compte d'une condition de sélection et sélection d'instants caractéristiques de la fonction temporelle sinusoïdale, résultant de div), chaque instant caractéristique correspondant à un instant auquel ladite fonction temporelle sinusoïdale satisfait à la condition de sélection ;
- dvi) à partir des instants caractéristiques de la fonction temporelle sinusoïdale, sélection d'instants caractéristiques de la composante pulsatile, chaque instant caractéristique de la composante pulsatile correspondant à un instant auquel ladite composante pulsatile satisfait à la condition de sélection ;
- dvii) détermination de la fréquence ou de la période de l'évolution temporelle du paramètre physiologique à partir des instants caractéristiques de la composante pulsatile résultant de dvi).

Selon un mode de réalisation :

- diii) comporte un filtrage passe bande de l'expression fréquentielle résultant de di), dans une bande passante comportant la fréquence dominante sélectionnée lors de dii), de façon à obtenir une expression fréquentielle filtrée ;
- div) comporte un calcul d'une expression temporelle de l'expression fréquentielle filtrée, l'expression temporelle calculée correspondant à la fonction sinusoïdale.

Selon une possibilité,

- dans dv), la condition de sélection est l'atteinte d'un extremum, l'extremum étant soit un maximum, soit un minimum, chaque instant caractéristique étant un extremum de la fonction sinusoïdale ;
- dans dvi), chaque instant caractéristique est un extremum de la composante pulsatile.

Selon une autre possibilité, la condition de sélection peut être un passage par une valeur prédéterminée, par exemple un passage par zéro.
L'étape dvi) peut comporter :

- définition d'un intervalle temporel autour de chaque instant caractéristique sélectionné à partir de la fonction temporelle sinusoïdale, l'intervalle temporel étant plus court qu'une période de la fonction temporelle sinusoïdale ;
- dans chaque intervalle temporel, sélection d'un instant auquel la composante pulsatile satisfait à la condition de sélection.

Selon un mode de réalisation :

- l'étape div) met en œuvre un algorithme de déformation temporelle dynamique, de façon à optimiser un appariement entre la fonction sinusoïdale et la composante pulsatile ;
- dans dvi), les instants caractéristiques de la composante pulsatile correspondent aux instants caractéristiques de la fonction sinusoïdale.

Selon un mode de réalisation, dvii) comporte une estimation d'une fréquence ou d'une période cardiaque. La fréquence cardiaque ou la période cardiaque peut être estimée à partir de durées séparant des instants caractéristiques successifs de la composante pulsatile. L'étape dvii) peut comporter :

- un calcul d'une évolution temporelle de la fréquence ou de la période cardiaque ;
- une détermination d'au moins une période de l'évolution temporelle de la fréquence ou de la période cardiaque ;
- une estimation d'une période ou d'une fréquence respiratoire à partir de la période de ladite évolution temporelle.

L'étape dvii) peut comporter une application d'un lissage à l'évolution temporelle du rythme cardiaque, préalablement à la détermination de la période ou de la fréquence respiratoire. Selon un mode de réalisation, le procédé peut alors comporter une étape de caractérisation de l'activité cardiaque ou respiratoire, comprenant :

- formation d'une ligne de base, passant par la fonction temporelle de mesure ou la composante pulsatile à chaque instant caractéristique résultant de dvi) ;
- obtention d'une fonction de caractérisation, par soustraction de la fonction temporelle de mesure ou de la composante pulsatile à la ligne de base, la fonction de caractérisation comportant des lobes, chaque lobe s'étendant entre deux instants caractéristiques successifs ;
- caractérisation de l'activité cardiaque ou respiratoire à partir d'une forme ou d'une surface des lobes de la fonction de caractérisation.

Avantageusement, selon ce mode de réalisation, la condition de sélection prise en compte lors de dv) et dvi) est l'atteinte d'un extremum.
Selon un mode de réalisation :

- dans l'étape a), le détecteur est un photodétecteur ;
- l'étape a) comporte une application d'un dispositif optique contre une zone corporelle (2) de l'utilisateur , le dispositif optique comportant :

  • une source de lumière agencée pour émettre une lumière vers la zone corporelle ;
  • le photodétecteur, agencé pour détecter une intensité d'une lumière émise par la source de lumière et s'étant propagée dans la zone corporelle ;

- l'étape a) comporte une illumination de la zone corporelle par la source de lumière et une mesure de l'intensité détectée par le photodétecteur en différents instants ;
- dans l'étape b), la fonction de mesure correspond à une évolution temporelle de l'intensité détectée.

Selon un mode de réalisation :

- dans l'étape a), le détecteur est un détecteur acoustique ;

- l'étape a) comporte une disposition d'un dispositif acoustique contre une zone corporelle de l'utilisateur , le dispositif acoustique comportant :

  • une source acoustique, agencée pour émettre une onde ultrasonore vers la zone corporelle;
  • le détecteur acoustique, agencé pour détecter une onde acoustique réfléchie par la zone corporelle ;

- l'étape a) comporte une sonication (exposition aux ultrasons) de la zone corporelle par la source acoustique et une mesure de l'onde acoustique réfléchie la zone corporelle en différents instants ;
- dans l'étape b), la fonction de mesure correspond à une évolution temporelle de l'onde acoustique mesurée.

Selon un mode de réalisation,

- dans l'étape a), le détecteur est un détecteur de pression;
- l'étape a) comporte une disposition du détecteur de pression contre une zone corporelle de l'utilisateur ;
- l'étape a) comporte une mesure de la pression exercée par la zone corporelle en différents instants ;
- dans l'étape b), la fonction de mesure correspond à une évolution temporelle de la pression mesurée.

Un deuxième objet de l'invention est un dispositif pour déterminer une fréquence ou d'une période d'une évolution temporelle d'un paramètre physiologique, le paramètre physiologique variant de façon périodique en fonction du temps, sous l'effet d'une activité cardiaque ou respiratoire d'un utilisateur, le dispositif comportant:

- un détecteur, configuré pour mesurer un signal représentatif d'un paramètre physiologique de l'utilisateur en différents instants ;
- une unité de traitement, configurée pour mettre en œuvre les étapes b à d) d'un procédé selon le premier objet de l'invention à partir de signaux détectés par le détecteur aux différents instants.

Le dispositif peut comporter une source de lumière, agencée pour émettre une lumière vers une zone corporelle de l'utilisateur, le détecteur étant un photodétecteur, agencé pour détecter une intensité d'une lumière émise par la source de lumière et s'étant propagée dans la zone corporelle.

Le dispositif peut comporter une source acoustique, agencée pour émettre une onde ultrasonore vers une zone corporelle de l'utilisateur, le détecteur étant un détecteur acoustique, agencé pour détecter une onde acoustique réfléchie par la zone corporelle.

Le détecteur peut être un capteur de pression, agencé pour mesurer une pression exercée par la zone corporelle.

L'invention sera mieux comprise à la lecture de l'exposé des exemples de réalisation présentés, dans la suite de la description, en lien avec les figures listées ci-dessous.

## FIGURES

Les figures 1A et 1B schématisent un premier mode de réalisation d'un dispositif selon l'invention.

La figure 1C représente un autre mode de réalisation de dispositif selon l'invention.

La figure 2A représente un exemple de signaux PPG détectés, respectivement dans trois bandes spectrales.

La figure 2B est un détail de la figure 2A.

La figure 3 schématise les principales étapes d'un procédé selon l'invention.

La figure 4A montre les composantes pulsatile des signaux PPG représentés sur la figure 2B.

La figure 4B est un détail de la figure 4A.

La figure 4C est une expression fréquentielle de composantes pulsatiles, tels que représentées sur les figures 4A et 4B, chaque expression étant calculée selon une fenêtre temporelle correspondant à 256 échantillons.

La figure 4D montre une composante pulsatile du signal PPG, telle que représentée sur la figure 4A, ainsi qu'une fonction sinusoïdale obtenue à une fréquence dominante de la composante pulsatile.

La figure 4E est un détail de la figure 4D. LA figure 4E illustre une recherche de maxima dans la composante pulsatile à partir de maxima de la fonction sinusoïdale.

La figure 5A montre une évolution temporelle d'un rythme cardiaque estimé en mettant en œuvre les étapes schématisées sur la figure 3, à partir d'un signal PPG représenté sur la figure 2A.

La figure 5B est un détail de la figure 5A.

La figure 5C est obtenue par un lissage de la figure 5A.

La figure 5D est un détail de la figure 5C.

La figure 6A montre une fonction sinusoïdale obtenue par déformation temporelle dynamique. La figure 6B correspond à la figure 5B.

La figure 6C représente une évolution temporelle d'un rythme cardiaque, déterminé à partir de la figure 6A, dans un intervalle temporel identique à celui considéré pour la figure 6B.

La figure 7A montre un signal PPG sur lequel sont repérés des instants caractéristiques. On a également représenté, en pointillés, une ligne de base joignant le signal PPG entre les instants caractéristiques.

La figure 7B montre une fonction de caractérisation obtenue à partir du signal PPG et de la ligne de base représentés sur la figure 7A .

La figure 7C est un détail de la figure 7B.

## EXPOSE DE MODES DE REALISATION PARTICULIERS

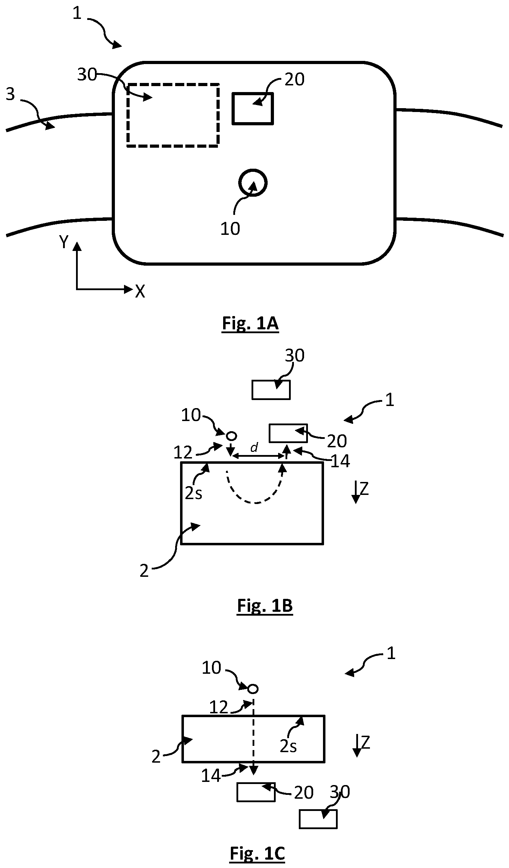

La figure 1A représente un exemple d'un dispositif 1 permettant une mise en œuvre de l'invention. Le dispositif comporte un détecteur 20, configuré pour détecter un signal représentatif d'un paramètre physiologique d'un utilisateur, en différents instants. L'utilisateur peut être un être humain ou un animal vivant. Le paramètre physiologique mesuré par le détecteur permet une estimation d'un rythme cardiaque ou d'un rythme respiratoire. Dans le mode de réalisation décrit en lien avec les figures 1A à 1C, le dispositif 1 est un dispositif optique. Dans un autre mode de réalisation, le dispositif 1 peut être un dispositif acoustique.

Le dispositif optique 1 est destiné à être appliqué contre une zone corporelle 2 de l'utilisateur. La zone corporelle peut par exemple être située au niveau d'un poignet ou d'un doigt de l'utilisateur. Dans l'exemple représenté, le dispositif 1 est relié à un lien 3, de type bracelet, de façon à être fixé contre un poignet. Le dispositif peut être appliqué contre toute autre zone corporelle suffisamment vascularisée : ventre, poitrine, lobe de l'oreille, cou, doigt, jambe, ces exemples n'étant pas limitatifs.

Le dispositif 1 comporte une source de lumière 10, configurée pour émettre un faisceau lumineux incident 12 vers la zone corporelle 2 face à laquelle la source de lumière est disposée. Le faisceau lumineux incident 12 se propageant vers la zone corporelle 2, selon un axe de propagation Z. Les photons du faisceau lumineux incident 12 pénètrent dans la zone corporelle 2 et une partie d'entre eux est rétrodiffusée, par exemple selon une direction parallèle à l'axe de propagation Z, dans un sens opposé à ce dernier. Les photons rétrodiffusés constituent un rayonnement rétrodiffusé 14. Le rayonnement rétrodiffusé 14 peut être détecté par un photodétecteur 20, placé en regard d'une surface 2s de la zone corporelle. Le photodétecteur 20 peut être configuré de façon à détecter un rayonnement rétrodiffusé émanant de la zone corporelle à une distance d, dite distance de rétrodiffusion, généralement non nulle et inférieure à quelques millimètres, typiquement inférieure à 15 mm ou 10 mm. Le photodétecteur 20 permet de mesurer l'intensité du rayonnement rétrodiffusé.

La source de lumière 10 peut être une LED (Light Emitting Diode - Diode électroluminescente), dont la bande spectrale d'émission s'étend dans le visible ou l'infra-rouge. De préférence, la largeur de la bande spectrale d'émission est inférieure à 100 nm. Le photodétecteur 20 peut être une photodiode. Le signal détecté par le photodétecteur est un signal de photoplethysmographie (PPG).

La figure 1B schématise les principaux composants du dispositif 1 dans un plan perpendiculaire à la surface 2s de la zone corporelle. La flèche courbe en pointillés représente un trajet optique de photons émis par la source de lumière, dans la zone corporelle analysée.

Le dispositif optique 1 comporte une unité de traitement 30 configurée pour traiter un signal détecté par le photodétecteur 20. L'unité de traitement 30 est reliée à une mémoire 32, dans laquelle sont stockées des instructions pour mettre en œuvre le procédé décrit par la suite. L'unité de traitement peut comporter un microprocesseur.

Selon une alternative, représentée sur la figure 1C, la zone corporelle 2 s'étend entre la source de lumière 10 et le photodétecteur 20. Selon une telle configuration, dite en transmission, le photodétecteur 20 mesure une intensité des photons ayant traversé la zone corporelle 2. Une telle configuration suppose que la zone corporelle soit suffisamment fine pour qu'une quantité suffisante de photons émane du milieu et soit détectée par le photodétecteur. Il peut par exemple s'agir du lobe d'une oreille ou du doigt.

Quel que soit le mode de réalisation, le photodétecteur 20 est agencé pour mesurer une intensité d'un faisceau de lumière formé par des photons s'étant propagés à travers la zone corporelle 2 : il s'agit soit de photons rétrodiffusés, soit de photons ayant traversé la zone corporelle. Dans la suite de la description, on se base sur la configuration en réflexion représentée sur la figure 1B, la zone corporelle étant un doigt d'un patient.

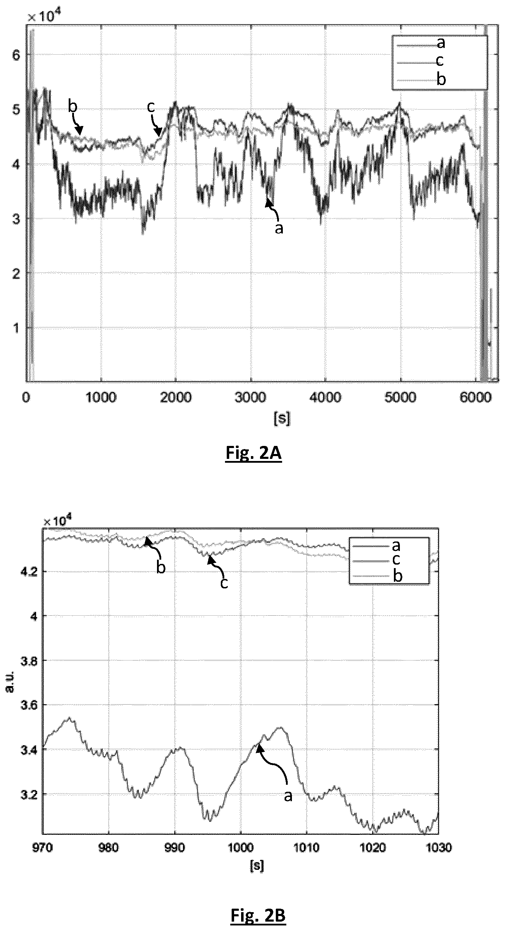

Les figures 2A et 2B montrent des exemples de signaux $PPG$ détectés par le photodétecteur suite à des illuminations d'une zone corporelle (doigt), en réflexion, respectivement dans trois bandes spectrales, respectivement centrées sur le vert (courbe a - $\lambda$ = 574 nm), le rouge (courbe b - $\lambda$ = 645 nm) et l'infra-rouge (courbe c - $\lambda$ = 940 nm). La figure 2A montre une fonction de mesure g, correspondant au signal $PPG$ mesuré pendant 6 000 secondes. La figure 2B est un détail de la figure 2A, sur une plage temporelle réduite, entre 970 s et 1030 s. Sur les figures 2A et 2B, l'axe des abscisses est le temps (s) et l'axe des ordonnées est l'intensité lumineuse détecté (unités arbitraires - a.u.). La fonction de mesure désigne les signaux mesurés par le photodétecteur en différents instants de mesure.

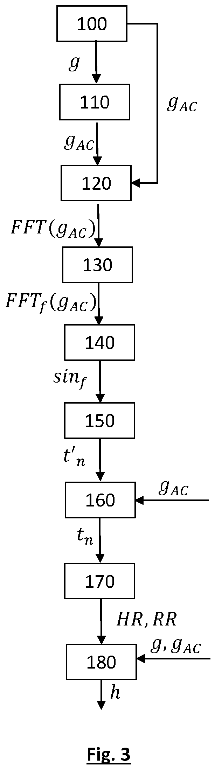

La figure 3 schématise les principales étapes permettant une estimation d'un intervalle inter-battements (ou période cardiaque), que l'on peut convertir en fréquence cardiaque (ou rythme cardiaque) HR. Le procédé comporte :

- une extraction d'une composante pulsatile $g_{AC}$ de la fonction de mesure $g$ : la composante pulsatile est une fonction périodique dont la période dépend du rythme cardiaque;
- une estimation de la fréquence cardiaque à partir de la composante pulsatile extraite, et cela sans seuillage en intensité de la composante pulsatile.

Les principales étapes du procédé sont à présent décrites.

Etape 100 : acquisition du signal *PPG.* Il s'agit d'acquérir un signal détecté par le photodétecteur 20 durant une plage temporelle déterminée. On obtient une fonction de mesure g correspondant au signal *PPG,* tel que représentée sur les figures 2A et 2B. La fréquence d'acquisition est de préférence supérieure à 10 Hz, par exemple 50 Hz.

Au cours de l'acquisition, la composante basse fréquence de la fonction de mesure g peut être éliminée par application d'un filtrage analogique passe-haut, de façon à supprimer les composantes fréquentielles du signal en dessous d'une fréquence de coupure, cette dernière étant de préférence inférieure à 0,5 Hz, par exemple 0.2 Hz. Dans ce cas, le signal issu du détecteur représente directement la composante pulsatile $g_{AC}$ du signal PPG.

Etape 110 : lorsque le filtrage passe-haut n'a pas été effectué de manière analogique lors de l'acquisition, l'étape 110 est mise en œuvre, dans l'unité de traitement 30, de façon à extraire une composante pulsatile de la fonction de mesure g. Cette étape revient à appliquer un filtre numérique passe haut. Elle peut être mise en œuvre en retirant de la fonction de mesure g, à un instant t, une moyenne de la fonction de mesure sur une durée prédéterminée, par exemple 1 seconde, et centrée sur l'instant t :

$$g_{AC}(t) = g(t) - \bar{g}(t) \quad (1)$$

$\bar{g}(t)$ est une moyenne de $g(t)$ calculée dans un intervalle temporel comportant l'instant *t,* et de préférence centré sur l'instant t.

L'étape 110 peut être mise en œuvre avec d'autres techniques de filtrage numérique, par exemple un filtre à réponse impulsionnelle finie, par exemple un filtre de Savitzky-Golay, ou encore un filtre à réponse impulsionnelle infinie. Elle permet d'extraire la composante pulsatile $g_{AC}$ à partir de la fonction de mesure g détectée par le photodétecteur 20.

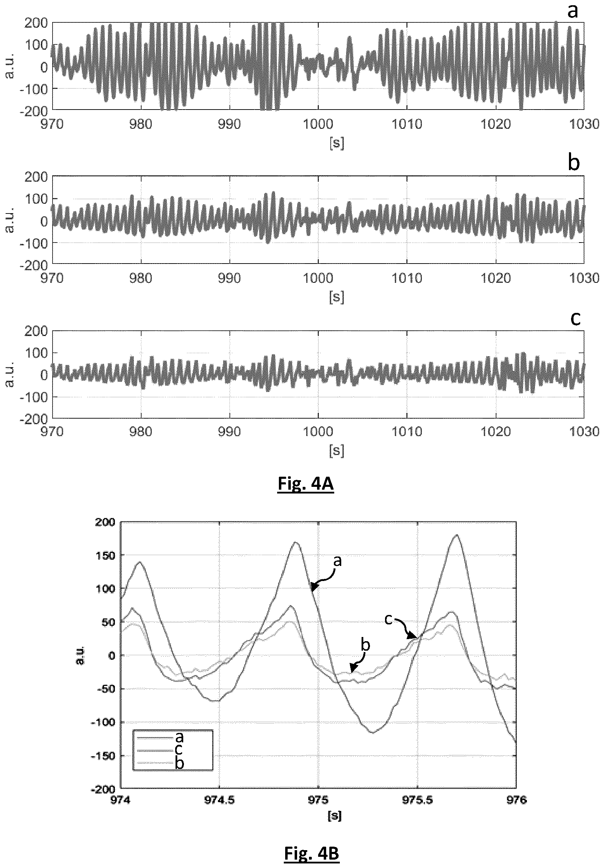

La figure 4A montre une composante pulsatile $g_{AC}$ extraite à partir de la fonction de mesure g représentée sur la figure 2B. Sur la figure 4A, on a représenté les fonctions de mesure dans les trois bandes spectrales précédemment évoquées : le vert (courbe a - $\lambda$ = 574 nm), le rouge (courbe b - $\lambda$ = 645 nm) et l'infra-rouge (courbe c - $\lambda$ = 940 nm). Sur la figure 4A, chaque axe des abscisses correspond au temps et chaque axe des ordonnées correspond à une unité arbitraire (a.u.). La figure 4B est un détail des courbes représentées sur la figure 4A, dans une plage temporelle réduite. On observe que les composantes pulsatiles extraites de fonctions de mesure g formées dans les trois bandes spectrales sont des fonctions périodiques, chaque période correspondant à un battement cardiaque. Les trois composantes représentées sur les figures 4A et 4B sont exploitables pour déterminer le rythme cardiaque. Toutefois, dans la bande spectrale verte, la dynamique est plus élevée que dans les bandes spectrales rouge ou infra-rouge. La bande spectrale verte est donc préférée. Dans la suite de la description, on se limite à la composante acquise dans la bande spectrale verte, sachant que le procédé peut s'appliquer dans les autres bandes spectrales, dans le domaine visible ou infra-rouge.

La composante pulsatile $g_{AC}$ est une fonction périodique, dont la période dépend du rythme cardiaque. Chaque maximum de la composante pulsatile $g_{AC}$ correspond à un instant auquel le volume de sang, dans la zone corporelle illuminée, est minimum. La décroissance suivant chaque maximum correspond à la systole cardiaque.

Etape 120 : expression fréquentielle de la composante pulsatile.

Au cours de l'étape 120, on applique une transformée de Fourier à la composante pulsatile $g_{AC}$. Cela permet d'obtenir une expression fréquentielle $FFT(g_{AC})$ de la composante pulsatile $g_{AC}$. La transformée de Fourier est calculée en appliquant une fenêtre temporelle glissante $\Delta$t de quelques secondes, par exemple un peu plus 5 secondes, soit 256 échantillons si l'on prend en compte la fréquence d'acquisition de 50 Hz évoquée dans l'étape 100. D'autres types de transformées permettant d'obtenir une expression fréquentielle de g sont envisageables.

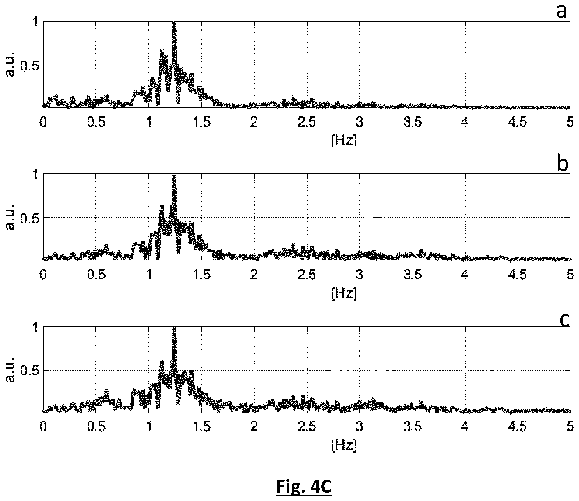

La figure 4C représente une expression fréquentielle $FFT(g_{AC})$ calculée, dans une fenêtre temporelle de 256 échantillons, à partir de fonctions telles qu'illustrées sur les figures 4A et 4B. courbe a : $\lambda$ = 574 nm ; courbe b : $\lambda$ = 645 nm ; courbe c : $\lambda$ = 940 nm. Sur la figure 4C, l'axe des abscisses correspond à la fréquence (unité Hz) et l'axe des ordonnées correspond à la puissance spectrale (unité arbitraire).

Etape 130 : sélection d'une fréquence dominante.

Au cours de l'étape 130, on sélectionne la fréquence f à laquelle la puissance spectrale de l'expression fréquentielle $FFT(g_{AC})$ est maximale. La fréquence sélectionnée est une fréquence dominante. Les autres fréquences sont annulées. On obtient ainsi une expression fréquentielle $FFT_f(g_{AC})$ filtrée selon la fréquence dominante, c'est-à-dire restreinte à la fréquence dominante.

Etape 140 : calcul d'une fonction temporelle sinusoïdale à partir de la fréquence dominante sélectionnée.

Au cours de cette étape, on effectue une transformée de Fourier inverse de l'expression fréquentielle $FFT_f(g_{AC})$, restreinte à la fréquence dominante *f* résultant de l'étape 130. On obtient une fonction sinusoïdale temporelle $sin_f(t) = FFT^{-1}_f(g_{AC})$, alignée à la composante pulsatile $g_{Ac}$. Par aligné, on entend que les maxima et les minima de la composante pulsatile $g_{AC}$ sont, à une incertitude près, en coïncidence temporelle avec les maximas et minimas de la fonction sinusoïdale temporelle $sin_f$.

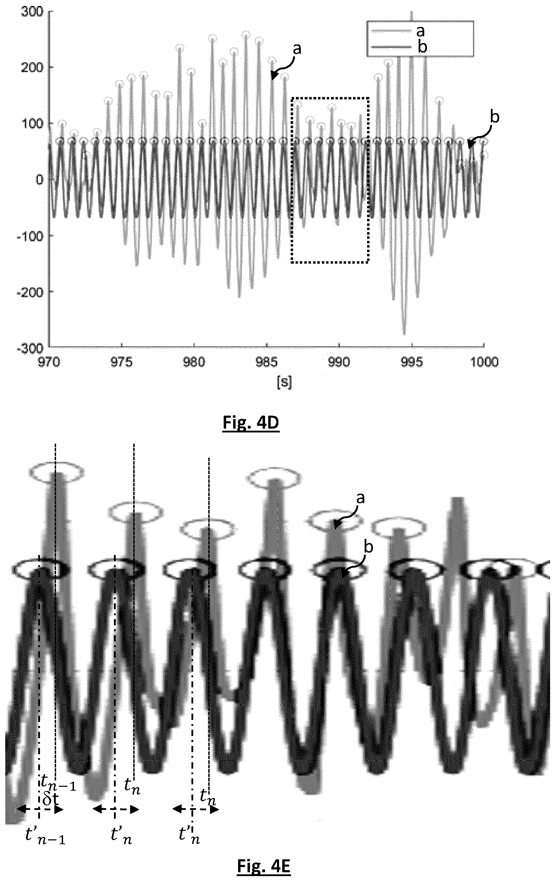

La figure 4D représente la composante pulsatile $g_{AC}$ (en gris - courbe a) ainsi que la fonction sinusoïdale $sin_f$ en noir (courbe b). La figure 4E est un détail de la figure 4D, correspondant au cadre en pointillés tracé sur la figure 4D. Sur les figures 4D et 4E, l'axe des abscisses est le temps et l'axe des ordonnées est la valeur de chaque fonction. Les maxima de chaque fonction sont encerclés.

On observe que les minima et les maxima de la composante pulsatile $g_{AC}$ de la fonction sinusoïdale $sin_f$ sont grossièrement en coïncidence temporelle mais apparaissent selon un léger décalage temporel. La figure 4E permet d'identifier certains décalages temporels. On observe également que le décalage n'est pas constant. La composante pulsatile est parfois « en avance » par rapport à la fonction sinusoïdale, et parfois en retard. Il y a donc un certain déphasage temporel entre la fonction sinusoïdale et la composante pulsatile $g_{Ac}$.

Comme on peut le voir sur les figures 4D et 4E, il y a un déphasage temporel variable entre la fonction sinusoïdale $sin_f(t)$ et la composante pulsatile $g_{AC}(t)$. Le déphasage temporel traduit le fait que la fréquence cardiaque HR n'est pas constante mais fluctue au cours du temps. Ce phénomène est usuellement désigné RSA (Respiratory Sinus Arrhythmia - Arythmie respiratoire sinusale) : la fréquence cardiaque tend à augmenter durant une inspiration, et à diminuer lors d'une expiration

Etape 150. sélection d'instants caractéristiques.

Au cours de cette étape, on sélectionne des instants caractéristiques $t'_n$ de la fonction sinusoïdale $sin_f(t)$, à partir desquels on sélectionne des instants caractéristiques $t_n$ de la composante pulsatile $g_{AC}(t)$. Les instants caractéristiques satisfont à une condition de sélection prédéterminée. Dans cet exemple, la condition de sélection est que l'instant caractéristique correspond à un maximum local. Selon d'autres possibilités, la condition de sélection est que l'instant caractéristique passe par un minimum local ou passe par une valeur prédéterminée, par exemple 0. L'indice n est un entier assigné chronologiquement à chaque instant chronologique $t'_n$ ou $t_n$.

Il est aisé de déterminer des d'instants $t'_n$ correspondant à des maxima (ou minima) de la fonction sinusoïdale $sin_f$. Au cours de l'étape 150, à partir de chaque instant $t'_n$ sélectionné sur la fonction sinusoïdale $sin_f$, on sélectionne un instant caractéristique $t_n$ sur la composante pulsatile $g_{AC}$, satisfaisant la condition de sélection, en l'occurrence être un maximum local.

Un aspect important de l'invention est que l'on tire profit de la fonction sinusoïdale $sin_f$, de fréquence $f$, résultant de l'étape 140, pour sélectionner les instants caractéristiques sur la composante pulsatile $g_{Ac}$. Chaque maximum de la fonction sinusoïdale est considéré comme proche d'un maximum de la composante pulsatile $g_{AC}$. Au cours de l'étape 150, un intervalle temporel $\delta t$ est défini autour de chaque instant caractéristique $t'_n$

correspondant à un maximum de la fonction sinusoïdale $sin_f$. Chaque intervalle temporel $\delta t$ est illustré par une double flèche sur la figure 4E. Une recherche d'un maximum local de la composante pulsatile $g_{AC}$ est effectuée dans chaque intervalle temporel $\delta t$. On observe que le maximum local de la composante pulsatile $g_{AC}$ est situé à un instant $t_n$ antérieur ou postérieur à l'instant $t'_n$ du maximum local de la composante sinusoïdale $sin_f$.

La durée de chaque intervalle temporel $\delta t$ est soit fixe, soit adaptative, en dépendant de la fréquence $f$ de la fonction sinusoïdale $sin_f$. La durée de chaque intervalle temporel $\delta t$ est inférieure à la période $\dfrac{1}{f}$ et de préférence à la demi période $\dfrac{1}{2f}$ de la fonction sinusoïdale $sin_f$.

Par exemple, la durée de chaque intervalle temporel $\delta t$ est $\dfrac{2}{3f}$ ce qui correspond à un nombre d'intensités mesurées égal à $round\left(\dfrac{2N}{3 \times f}\right)$, $N$ étant le nombre d'échantillons durant 1 seconde, correspondant à la fréquence d'échantillonnage, soit 50, $round$ désignant l'opérateur « entier le plus proche ».

La fonction sinusoïdale $sin_f$ définit une fréquence moyenne $f$, durant la fenêtre glissante $\Delta t$ prise en compte lors de l'étape 120 pour calculer l'expression fréquentielle $FFT(g_{AC})$. Les instants caractéristiques $t'_n$ sélectionnés sur la fonction sinusoïdale $sin_f$ sont régulièrement répartis selon la fréquence moyenne.

Etape 160

Au cours de l'étape 160, les instants $t_n$ caractéristiques sélectionnés sont utilisés pour déterminer, à chaque instant $t_n$, un intervalle inter-battements $IBI(t_n)$, (qui correspond à une période cardiaque) avec :

$$IBI(t_n) = t_{n+1} - t_n \quad (2)$$

La fréquence cardiaque (ou rythme cardiaque HR) correspond à $\dfrac{1}{IBI(t_n)}$ exprimée en Hz.

Etape 170

Au cours de l'étape 170, la fonction temporelle $HR(t)$ correspondant à l'évolution temporelle du rythme cardiaque peut faire l'objet d'un lissage, par exemple au moyen d'un filtre médian centré sur chaque instant $t_n$ et s'étendant sur trois instants successifs $t_{n-1}$, $t_n$ et $t_{n+1}$. Cette étape est optionnelle. Elle permet de corriger certaines erreurs de mesure.

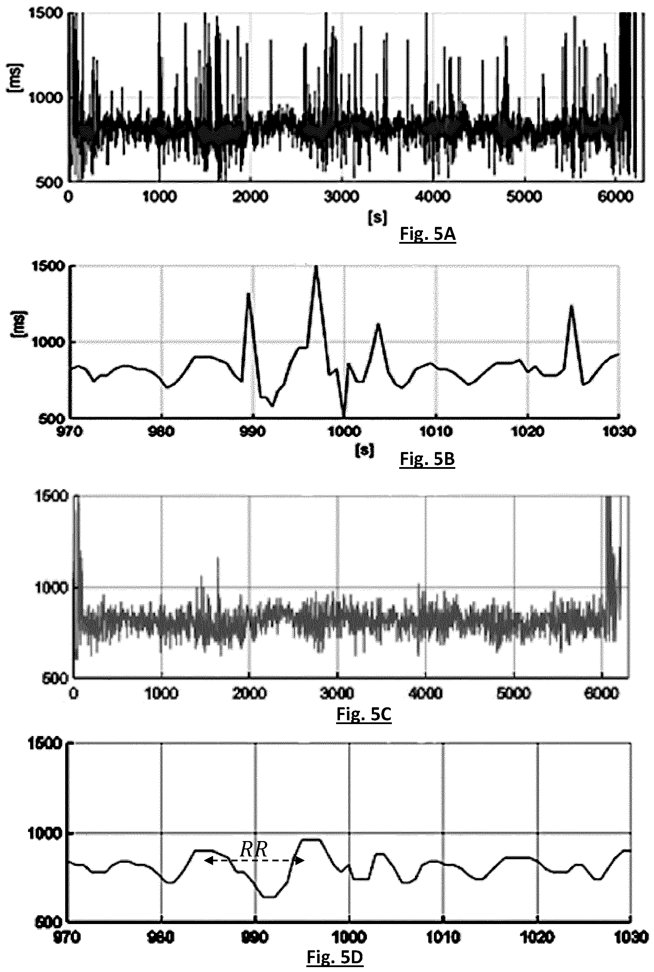

La figure 5A montre la période cardiaque (axe des ordonnées - unité : ms), en fonction du temps (axe des abscisses - unité : s), obtenue en mettant en œuvre le procédé précédemment décrit sur le signal PPG représenté sur la figure 2A, préalablement à un lissage. La figure 5B est un détail de la figure 5A, montrant la période cardiaque correspondant au signal PPG représenté sur la figure 2B.

La figure 5C (mêmes unités que figure 5A) résulte d'une application d'un lissage, tel que décrit en lien avec l'étape 170, sur l'évolution temporelle de la période cardiaque représentée sur la figure 5A. La figure 5D est un détail de la figure 5C, établi à partir du signal PPG représenté sur la figure 2B. Les fluctuations périodiques observées sur la figure 5B correspondent au rythme respiratoire RR. Ainsi, la méthode permet non seulement d'estimer la période (ou la fréquence) cardiaque, mais également le rythme respiratoire, en fonction du temps. Et cela avec un dispositif simple, porté au bout du doigt ou pouvant être intégré à une montre grand public.

Le procédé décrit en lien avec les étapes 100 à 170 peut être mis en œuvre simultanément en utilisant différentes bandes spectrales, par exemple centrées dans le vert (bande spectrale préférée), et/ou le rouge et/ou l'infra-rouge. Cela permet d'obtenir, de façon indépendante une estimation du rythme cardiaque et éventuellement du rythme respiratoire RR, dans différentes bandes spectrales. Les estimations dans chaque bande spectrale peuvent être ensuite combinées. On peut également sélectionner l'estimation considérée comme étant la plus proche d'une estimation à un instant précédent. Selon une possibilité, on sélectionne la bande spectrale en fonction de la puissance spectrale de l'expression fréquentielle $FFT(g_{AC})$ à la fréquence dominante $f$. Par exemple, on peut calculer, dans chaque bande spectrale, un ratio entre la puissance spectrale de l'expression fréquentielle $FFT(g_{AC})$ à la fréquence dominante $f$ et la puissance spectrale de l'ensemble des fréquences auxquelles $FFT(g_{AC})$ est déterminée. On retient la bande spectrale pour laquelle le ratio est le plus élevé.

Selon une variante, lors de l'étape 120, l'expression fréquentielle $FFT_f(g_{AC})$ de la composante pulsatile est obtenue par un chevauchement de 50% de deux fenêtres temporelles successives Δt. Cela permet qu'un même instant caractéristique $t_n$ de la composante pulsatile $g_{AC}$ soit identifié deux fois, respectivement à partir de expressions fréquentielles $FFT_f(g_{AC})$ formées à partir de deux fenêtres temporelles successives. On peut considérer la moyenne des instants caractéristiques détectés, ou sélectionner uniquement les instants caractéristiques $t_n$ détectés deux fois. Cela améliore la robustesse de l'estimation.

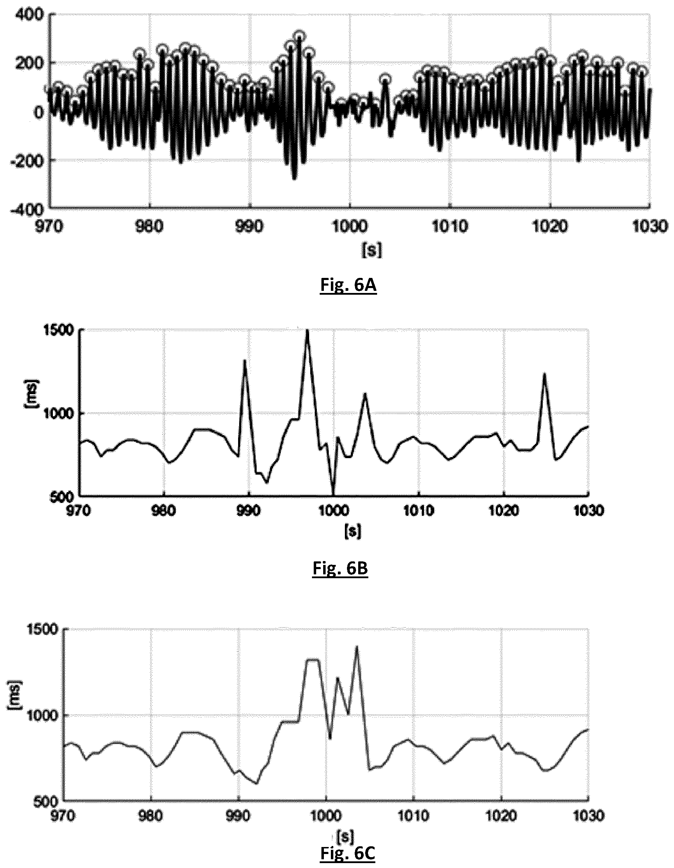

Selon une variante, lors de l'étape 140, la fonction sinusoïdale $sin_f$ est déformée en mettant en œuvre un algorithme de déformation temporelle dynamique, usuellement désigné par l'acronyme DTW (Dynamic Time Warping). La fonction sinusoïdale $sin_f$ est ainsi modifiée de façon à optimiser un appariement avec la composante pulsatile $g_{Ac}$. Selon cette variante, les instants caractéristiques $t'_n$ de la fonction sinusoïdale $sin_f$ sont considérés comme confondus avec les instants caractéristiques $t_n$ de la composante pulsatile $g_{AC}$. La figure 6A représente un exemple de sélection d'instants caractéristiques $t_n$ sur la composante pulsatile. Les figures 6B et 6C représentent respectivement les périodes cardiaques résultant :

- de l'étape 140, telle que décrite en lien avec la figure 5B ;
- de la variante de l'étape 140, mettant en œuvre l'algorithme DTW.

Sur la figure 6A, l'axe des abscisses correspond au temps (unité : seconde) et l'axe des ordonnées correspond à la valeur de la composante pulsatile (unité arbitraire). Sur les figures 6B et 6C, l'axe des abscisses correspond au temps (unité : seconde). L'axe des ordonnées correspond à la période cardiaque : unité milliseconde.

On observe une certaine similitude dans l'estimation de la période cardiaque, en dépit de certains disparités. Un inconvénient lié à l'algorithme DTW est une mémoire et une consommation plus importantes que l'étape 140 précédemment décrite.

Selon une variante, on peut effectuer un test de validité de chaque estimation de la période cardiaque $IBI(t_n)$ ou d'une fréquence cardiaque $HR(t_n)$. Il peut s'agir de vérifier que la période cardiaque (ou la fréquence cardiaque) est comprise dans un intervalle de validité. Lorsqu'il s'agit de la fréquence cardiaque, l'intervalle de validité peut être compris entre 40 et 200 bpm (battements par minute). En alternative ou en complément, le test de validité consiste à vérifier que l'intervalle inter-battements (ou la fréquence cardiaque) est compris dans un intervalle prédéterminé autour de l'intervalle inter-battements (ou de la fréquence cardiaque) estimé lors d'un instant précédent $t_{n-1}$. En fonction du test de validité, un indicateur de validité peut être assigné à chaque estimation $IBI(t_n)$ ou $HR(t_n)$ à l'instant $t_n$.

Selon une possibilité, le procédé peut comporter une étape 180 de caractérisation de l'activité cardiaque. Pour cela, les instants caractéristiques $t_n$ résultants de l'étape 160 sont reportés sur la fonction de mesure g ou sur la composante pulsatile $g_{AC}$. Une ligne de base BL est tracée entre la valeur de la fonction g ou de la composante pulsatile $g_{AC}$, à chaque instant caractéristique $t_n$. La fonction de mesure g, ou sa composante pulsatile $g_{AC}$, est ensuite soustraite à la ligne de base, de façon à obtenir une fonction de caractérisation h. La fonction de caractérisation comporte des lobes, chaque lobe représentant un volume sanguin entre deux pulsations cardiaques successives. Chaque lobe s'étend entre deux instants caractéristiques successifs $t_n$, $t_{n+1}$. Cela permet une caractérisation plus poussée de l'activité cardiaque, par l'aire et ou la forme de chaque lobe. De préférence, cette étape est mise en œuvre lorsque les instants caractéristiques $t_n$ correspondent à des maxima ou des minima de la fonction de mesure g ou sur la composante pulsatile $g_{AC}$.

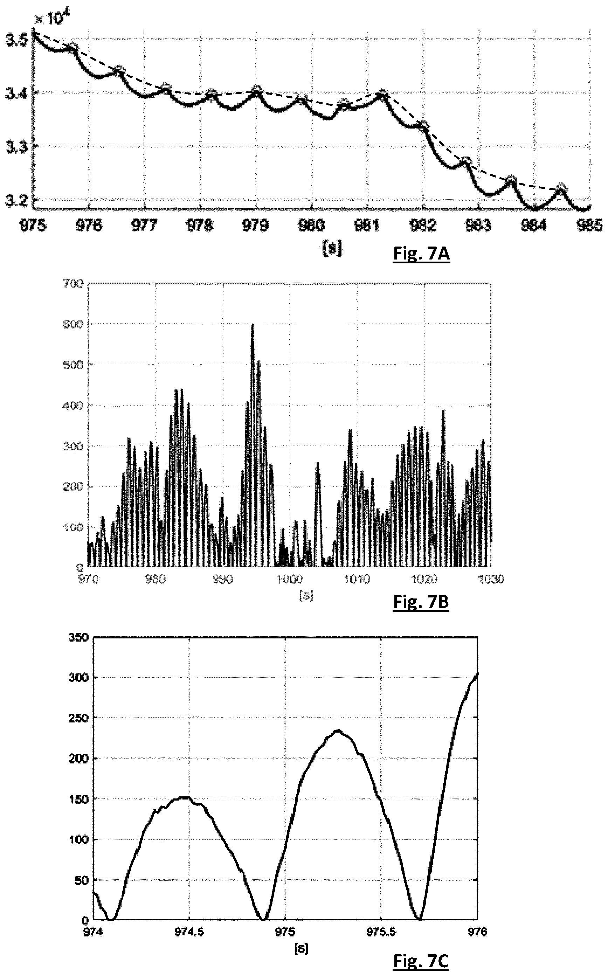

Les figures 7A, 7B et 7C représentent respectivement :

- un détail de la fonction de mesure décrite en lien avec la figure 2A (courbe en trait plein), ainsi qu'une ligne de base *BL*, passant par chaque instant caractéristique (courbe en pointillés) ;
- une fonction de caractérisation *h*, telle *que h = BL - g* ;
- un détail de la fonction de caractérisation h, montrant deux lobes séparés : on comprend que la fonction de caractérisation h permet d'accéder à une information plus poussée quant à l'activité cardiaque de l'utilisateur.

Sur les figures 7A, 7B et 7C, les unités des axes des ordonnées sont des unités arbitraires. L'axe des abscisses correspond au temps (unité : seconde).

Bien que décrite en lien avec un dispositif optique 1, l'invention peut s'appliquer à d'autres types de détecteurs, par exemple un détecteur acoustique, en particulier dans le domaine des ultrasons. On sait que l'échographie cardiaque peut également permettre d'accéder à des informations relatives à des variations pulsatiles de volumes sanguins. L'invention peut être appliquée à partir d'un signal d'échographie, par exemple une échographie Doppler. Le dispositif comporte au moins une source ultrasonore 10' et au moins un capteur acoustique 20'. Dans la pratique, on utilise généralement des transducteurs agissant à la fois en tant que source et détecteur. La fonction temporelle détectée g peut être un signal d'amplitude ou un signal de phase ou un signal de fréquence acoustique (spectrogramme). Chacun de ces signaux comporte une composante pulsatile périodique $g_{AC}$, dont la période dépend du rythme cardiaque. Les étapes 110 à 170, voire 180, précédemment décrites avec un signal optique, peuvent s'appliquer de la même manière, de façon à extraire des instants caractéristiques $t_n$ de la composante pulsatile $g_{AC}$, de façon à estimer le rythme cardiaque ou le rythme respiratoire.

L'invention peut également s'appliquer à l'échographie fœtale. Dans ce cas, chaque source et chaque détecteur ultrasonore est appliquée contre le fœtus, mais pas directement au contact de ce dernier, mais contre le ventre de la mère, ce dernier formant un milieu de propagation entre le dispositif et le fœtus.

L'invention peut également s'appliquer à un dispositif de type tonomètre comportant un capteur de pression appliqué au contact d'une zone corporelle.

L'invention peut également s'appliquer à des mesures de débit d'air expiré ou inspiré par un utilisateur, l'objectif étant de caractériser une activité respiratoire. Le débit d'air comporte une composante pulsatile $g_{AC}$, dont la période dépend du rythme respiratoire de l'utilisateur. Les étapes 110 à 170, voire 180, précédemment décrites avec un signal optique, peuvent s'appliquer de la même manière, de façon à extraire des instants caractéristiques $t_n$ de la composante pulsatile $g_{AC}$, de façon à estimer le rythme respiratoire et/ou caractériser l'activité respiratoire.

L'invention peut également s'appliquer, de façon plus générale, à une mesure de tout paramètre physiologique variant de façon périodique sous l'effet d'une activité cardiaque ou respiratoire, notamment lorsque l'évolution temporelle du paramètre physiologique peut être considérée comme sinusoïdale.

Le procédé est relativement simple à mettre en œuvre. Il est paramétré par :

- la fréquence d'échantillonnage des signaux détectés par le détecteur ;
- la durée $\Delta t$ de la fenêtre temporelle utilisée pour calculer une expression fréquentielle de la composante pulsatile $g_{AC}$ de la fonction de mesure g ;
- la durée $\delta t$ des intervalles temporels dans lesquels on effectue une recherche d'instants caractéristiques de la composante pulsatile à partir des instants caractéristiques de la fonction sinusoïdale $sin_f$

Le procédé est simple à mettre en œuvre et peut être intégré dans des dispositifs nomades, par exemple des montres ou des équipements médicaux portables.

## Revendications

1. Procédé de détermination d'une fréquence ou d'une période d'une évolution temporelle d'un paramètre physiologique, le paramètre physiologique variant de façon périodique en fonction du temps, sous l'effet d'une activité cardiaque ou respiratoire d'un utilisateur, le procédé comportant les étapes suivantes :

    a) application d'un dispositif optique (1) contre une zone corporelle (2) de l'utilisateur , le dispositif optique comportant :

    • une source de lumière (10), agencée pour émettre une lumière vers la zone corporelle ;
    • un photodétecteur (20), agencé pour détecter une intensité d'une lumière émise par la source de lumière et s'étant propagée dans la zone corporelle ;

    puis illumination de la zone corporelle par la source de lumière et mesure de l'intensité détectée par le photodétecteur en différents instants, l'intensité détectée formant un signal représentatif du paramètre physiologique de l'utilisateur aux différents instants ;
    b) à partir des signaux détectés, obtention d'une fonction de mesure (g), correspondant à une évolution temporelle de l'intensité détectée ;

c) obtention d'une composante pulsatile ($g_{AC}$) de la fonction de mesure, la composante pulsatile étant une fonction périodique dont la période dépend de l'activité cardiaque ou de l'activité respiratoire ;

d) à partir de la composante pulsatile, détermination du rythme cardiaque (HR) ou du rythme respiratoire (*RR*) ;

le procédé étant **caractérisé en ce que** l'étape d) comporte :

- di) obtention d'une expression fréquentielle ($FFT(g_{AC})$) de la composante pulsatile ;
- dii) sélection d'une fréquence dominante (*f*) de l'expression fréquentielle ;
- diii) filtrage de l'expression fréquentielle à la fréquence dominante, de façon à obtenir une expression fréquentielle filtrée ($FFT_f(g_{AC})$);
- div) à partir de l'expression fréquentielle filtrée, obtention d'une fonction temporelle sinusoïdale ($sin_f$) à la fréquence dominante ;
- dv) prise en compte d'une condition de sélection et sélection d'instants caractéristiques ($t'_n$) de la fonction temporelle sinusoïdale, résultant de div), chaque instant caractéristique correspondant à un instant auquel ladite fonction temporelle sinusoïdale ($sin_f$) satisfait à la condition de sélection ;
- dvi) à partir des instants caractéristiques ($t'_n$) de la fonction temporelle sinusoïdale, sélection d'instants caractéristiques ($t_n$) de la composante pulsatile, chaque instant caractéristique de la composante pulsatile correspondant à un instant auquel ladite composante pulsatile ($g_{AC}$) satisfait à la condition de sélection ;
- dvii) détermination de la fréquence ou de la période de l'évolution temporelle du paramètre physiologique à partir des instants caractéristiques ($t_n$) de la composante pulsatile résultant de dvi).

2. Procédé selon la revendication 1, dans lequel :

- diii) comporte un filtrage passe bande de l'expression fréquentielle résultant de di), dans une bande passante comportant la fréquence dominante sélectionnée lors de dii), de façon à obtenir une expression fréquentielle filtrée ;
- div) comporte un calcul d'une expression temporelle de l'expression fréquentielle filtrée ($FFT^{-1}_f(g_{AC})$), l'expression temporelle calculée correspondant à la fonction sinusoïdale ($sin_f$).

3. Procédé selon l'une quelconque des revendications précédentes, dans lequel :

- dans dv), la condition de sélection est l'atteinte d'un extremum, l'extremum étant soit un maximum, soit un minimum, chaque instant caractéristique ($t'_n$) étant un extremum de la fonction sinusoïdale ($sin_f$);
- dans dvi), chaque instant caractéristique ($t_n$) est un extremum de la composante pulsatile ($g_{AC}$).

4. Procédé selon l'une quelconque des revendications précédentes, dans lequel l'étape dvi) comporte :

- définition d'un intervalle temporel ($\delta t$) autour de chaque instant caractéristique sélectionné à partir de la fonction temporelle sinusoïdale, l'intervalle temporel étant plus court qu'une période de la fonction temporelle sinusoïdale ;
- dans chaque intervalle temporel, sélection d'un instant ($t_n$) auquel la composante pulsatile satisfait à la condition de sélection.

5. Procédé selon l'une quelconque des revendications 1 à 3, dans lequel :

- l'étape div) met en œuvre un algorithme de déformation temporelle dynamique, de façon à optimiser un appariement entre la fonction sinusoïdale ($sin_f$) et la composante pulsatile ($g_{AC}$);
- dans dvi), les instants caractéristiques ($t_n$) de la composante pulsatile ($g_{AC}$) correspondent aux instants caractéristiques (*t'n*) de la fonction sinusoïdale ($sin_f$).

6. Procédé selon l'une quelconque des revendications précédentes, dans lequel l'étape dvii) comporte une estimation d'une fréquence ou d'une période cardiaque

7. Procédé selon la revendication 6, dans lequel la fréquence cardiaque ou la période cardiaque est estimée à partir de durées séparant des instants caractéristiques ($t_n$, $t_{n+1}$) successifs de la composante pulsatile ($g_{AC}$).

8. Procédé selon la revendication 7 dans lequel dvii) comporte :

- un calcul d'une évolution temporelle de la fréquence ou de la période cardiaque ($HR(t)$);
- une détermination d'au moins une période (T) de l'évolution temporelle de la fréquence ou de la période cardiaque ;
- une estimation d'une période ou d'une fréquence respiratoire (RR(t)) à partir de la période de ladite évolution temporelle.

9. Procédé selon la revendication 8, dans lequel l'étape dvii) comporte une application d'un lissage à l'évolution temporelle du rythme cardiaque, préalablement à la détermination de la période ou de la fréquence respiratoire.

10. Procédé selon l'une quelconque des revendications précédentes, dans lequel la condition de sélection prise en compte lors de dv) et dvi) est l'atteinte d'un extremum, le procédé comportant une étape de caractérisation de l'activité cardiaque ou respiratoire, comprenant :

- formation d'une ligne de base (*BL*), passant par la fonction temporelle de mesure (g) ou la composante pulsatile ($g_{AC}$), à chaque instant caractéristique $(t_n)$ résultant de dvi) ;
- obtention d'une fonction de caractérisation (h), par soustraction de la fonction temporelle de mesure ou de la composante pulsatile à la ligne de base, la fonction de caractérisation comportant des lobes, chaque lobe s'étendant entre deux instants caractéristiques successifs ;
- caractérisation de l'activité cardiaque ou respiratoire à partir d'une forme ou d'une surface des lobes de la fonction de caractérisation.

11. Dispositif pour déterminer une fréquence ou d'une période d'une évolution temporelle d'un paramètre physiologique, le paramètre physiologique variant de façon périodique en fonction du temps, sous l'effet d'une activité cardiaque ou respiratoire d'un utilisateur, le dispositif comportant:

- une source de lumière (10), agencée pour émettre une lumière vers une zone corporelle de l'utilisateur,
- un photodétecteur (20), agencé pour détecter une intensité d'une lumière émise par la source de lumière et s'étant propagée dans la zone corporelle, l'intensité détectée formant un signal représentatif du paramètre physiologique de l'utilisateur en différents instants ;
- une unité de traitement (30), configurée pour mettre en œuvre les étapes b à d) d'un procédé selon l'une quelconque des revendications précédentes à partir des signaux détectés par le détecteur aux différents instants.

**Patentansprüche**

1. Verfahren zur Bestimmung einer Frequenz oder einer Periode eines zeitlichen Verlaufs eines physiologischen Parameters, wobei der physiologische Parameter in Abhängigkeit von der Zeit, unter der Wirkung einer Herz- oder Atemaktivität eines Benutzers, periodisch variiert, wobei das Verfahren die folgenden Schritte umfasst:

a) Anlegen einer optischen Vorrichtung (1) an einen Körperbereich (2) des Benutzers, wobei die optische Vorrichtung umfasst:

• eine Lichtquelle (10), die dazu eingerichtet ist, ein Licht zu dem Körperbereich hin zu emittieren,
• einen Fotodetektor (20), der dazu eingerichtet ist, eine Intensität eines Lichts zu messen, das von der Lichtquelle emittiert wird und sich in dem Körperbereich ausgebreitet hat;
dann Beleuchten des Körperbereichs durch die Lichtquelle und Messen der von dem Fotodetektor zu verschiedenen Zeitpunkten detektierten Intensität, wobei die detektierte Intensität ein Signal bildet, das für den physiologischen Parameter des Benutzers zu den verschiedenen Zeitpunkten repräsentativ ist;

b) ausgehend von den detektierten Signalen, Erhalten einer Messfunktion (g), die einem zeitlichen Verlauf der detektierten Intensität entspricht;
c) Erhalten einer pulsatilen Komponente ($g_{AC}$) der Messfunktion, wobei die pulsatile Komponente eine periodische Funktion ist, deren Periode von der Herzaktivität oder von der Atemaktivität abhängt;
d) ausgehend von der pulsatilen Komponente, Bestimmen des Herzrhythmus (*HR*) oder des Atemrhythmus (*RR*);
wobei das Verfahren **dadurch gekennzeichnet ist, dass** der Schritt d) umfasst:

- di) Erhalten eines Frequenzausdrucks ($FFT(g_{AC})$) der pulsatilen Komponente;
- dii) Auswählen einer dominanten Frequenz (*f*) des Frequenzausdrucks;
- diii) Filtern des Frequenzausdrucks für die dominante Frequenz, so dass ein gefilterter Frequenzausdruck ($FFT_f(g_{Ac})$) erhalten wird;
- div) ausgehend von dem gefilterten Frequenzausdruck, Erhalten einer Sinus-Zeitfunktion ($sin_f$) für die dominante Frequenz;
- dv) Berücksichtigen einer Auswahlbedingung und Auswählen von charakteristischen Zeitpunkten ($t'_n$) der aus div) resultierenden Sinus-Zeitfunktion, wobei jeder charakteristische Zeitpunkt einem Zeitpunkt entspricht, zu dem die Sinus-Zeitfunktion ($sin_f$) die Auswahlbedingung erfüllt;
- dvi) ausgehend von den charakteristischen Zeitpunkten ($t'_n$) der Sinus-Zeitfunktion, Auswählen von charakteristischen Zeitpunkten ($t_n$) der pulsatilen Komponen-

te, wobei jeder charakteristische Zeitpunkt der pulsatilen Komponenten einem Zeitpunkt entspricht, zu dem die pulsatile Komponente ($g_{AC}$) die Auswahlbedingung erfüllt;

- dvii) Bestimmen der Frequenz oder der Periode des zeitlichen Verlaufs des physiologischen Parameters ausgehend von den aus dvi) resultierenden charakteristischen Zeitpunkten ($t_n$) der pulsatilen Komponente.

2. Verfahren nach Anspruch 1, wobei:

- diii) eine Bandpassfilterung des aus di) resultierenden Frequenzausdrucks in einem Durchlassband umfasst, das die bei dii) ausgewählte dominante Frequenz umfasst, so dass ein gefilterter Frequenzausdruck erhalten wird;
- div) ein Berechnen eines Zeitausdrucks des gefilterten Frequenzausdrucks ($FFT^{-1}{}_f(g_{AC})$) umfasst, wobei der berechnete Zeitausdruck der Sinusfunktion ($sin_f$) entspricht.

3. Verfahren nach einem der vorhergehenden Ansprüche, wobei:

- in dv) die Auswahlbedingung von einem Extremum erreicht wird, wobei das Extremum entweder ein Maximum oder ein Minimum ist, wobei jeder charakteristische Zeitpunkt ($t'_n$) ein Extremum der Sinusfunktion ($sin_f$) ist;
- in dvi) jeder charakteristische Zeitpunkt ($t_n$) ein Extremum der pulsatilen Komponente ($g_{AC}$) ist.

4. Verfahren nach einem der vorhergehenden Ansprüche, wobei der Schritt dvi) umfasst:

- Definition eines Zeitintervalls ($\delta t$) um jeden ausgehend von der Sinus-Zeitfunktion ausgewählten charakteristischen Zeitpunkt herum, wobei das Zeitintervall kürzer als eine Periode der Sinus-Zeitfunktion ist;
- in jedem Zeitintervall, Auswählen eines Zeitpunkts ($t_n$), zu dem die pulsatile Komponente die Auswahlbedingung erfüllt.

5. Verfahren nach einem der Ansprüche 1 bis 3, wobei:

- der Schritt div) einen Algorithmus zur dynamischen Zeitverzerrung ausführt, so dass ein Abgleich zwischen der Sinusfunktion ($sin_f$) und der pulsatilen Komponente ($g_{AC}$) optimiert wird;
- in dvi) die charakteristischen Zeitpunkte ($t_n$) der pulsatilen Komponente ($g_{AC}$) den charakteristischen Zeitpunkten ($t'_n$) der Sinusfunktion ($sin_f$) entsprechen.

6. Verfahren nach einem der vorhergehenden Ansprüche, wobei der Schritt dvii) ein Schätzen einer Herzfrequenz oder -periode umfasst.

7. Verfahren nach Anspruch 6, wobei die Herzfrequenz oder die Herzperiode ausgehend von Dauern geschätzt wird, die aufeinander folgende charakteristische Zeitpunkte ($t_n$, $t_{n+1}$) der pulsatilen Komponente ($g_{AC}$) trennen.

8. Verfahren nach Anspruch 7, wobei dvii) umfasst:

- ein Berechnen eines zeitlichen Verlaufs der Herzfrequenz oder -periode ($HR(t)$);
- ein Bestimmen mindestens einer Periode (T) des zeitlichen Verlaufs der Herzfrequenz oder -periode;
- ein Schätzen einer Atemperiode oder -frequenz ($RR(t)$) ausgehend von der Periode des zeitlichen Verlaufs.

9. Verfahren nach Anspruch 8, wobei der Schritt dvii) ein Anwenden einer Glättung auf den zeitlichen Verlauf des Herzrhythmus vor dem Bestimmen der Atemperiode oder -frequenz umfasst.

10. Verfahren nach einem der vorhergehenden Ansprüche, wobei die bei dv) und dvi) berücksichtigte Auswahlbedingung von einem Extremum erreicht wird, wobei das Verfahren einen Schritt des Charakterisierens der Herz- oder Atemaktivität umfasst, beinhaltend:

- Bilden einer Basislinie ($BL$), die durch die zeitliche Messfunktion (g) oder die pulsatile Komponente ($g_{AC}$) verläuft, bei jedem charakteristischen Zeitpunkt ($t_n$), der aus dvi) resultiert;
- Erhalten einer Charakterisierungsfunktion (h), durch Subtrahieren der zeitlichen Messfunktion oder der pulsatilen Komponente von der Basislinie, wobei die Charakterisierungsfunktion Keulen umfasst, wobei sich jede Keule zwischen zwei aufeinander folgenden charakteristischen Zeitpunkten erstreckt;
- Charakterisieren der Herz- oder Atemaktivität ausgehend von einer Form oder von einer Fläche der Keulen der Charakterisierungsfunktion.

11. Vorrichtung zur Bestimmung einer Frequenz oder einer Periode eines zeitlichen Verlaufs eines physiologischen Parameters, wobei der physiologische Parameter in Abhängigkeit von der Zeit, unter der Wirkung einer Herz- oder Atemaktivität eines Benutzers, periodisch variiert, wobei die Vorrichtung umfasst:

- eine Lichtquelle (10), die dazu eingerichtet ist, ein Licht zu einem Körperbereich des Benutzers hin zu emittieren,
- einen Fotodetektor (20), der dazu eingerichtet ist, eine Intensität eines Lichts zu messen, das von der Lichtquelle emittiert wird und sich in dem Körperbereich ausgebreitet hat, wobei die detektierte Intensität ein Signal bildet, das für den physiologischen Parameter des Benutzers zu verschiedenen Zeitpunkten repräsentativ ist;
- eine Verarbeitungseinheit (30), die dazu ausgestaltet ist, die Schritte b bis d) eines Verfahrens nach einem der vorhergehenden Ansprüche ausgehend von den von dem Fotodetektor zu den verschiedenen Zeitpunkten detektierten Signalen auszuführen.

**Claims**

1. A method for determining a frequency or period of a time-domain variation in a physiological parameter, the physiological parameter varying periodically as a function of time, under the effect of a cardiac activity or respiratory activity of a user, the method comprising:

   a) applying an optical device (1) against a bodily region (2) of the user, the optical device comprising :

      • a light source (10) arranged to emit light toward the bodily region;
      • a photodetector (20), arranged to detect an intensity of light emitted by the light source and having propagated through the bodily region;

         illuminating the bodily region with the light source;
         and measuring the intensity detected by the photodetector at various times ;

   b) on the basis of the detected intensities, obtaining a measurement function (g), which corresponds to a time-domain variation in detected intensity;
   c) obtaining a pulsed component ($g_{AC}$) of the measurement function, the pulsed component being a periodic function the period of which depends on the cardiac activity or respiratory activity;
   d) on the basis of the pulsed component, determining heart rate (HR) or breathing rate (RR); wherein d) comprises:

      - di) obtaining a frequency-domain expression ($FFT(g_{AC})$) for the pulsed component;
      - dii) selecting a dominant frequency (f) of the frequency-domain expression;

      - diii) filtering the frequency-domain expression at the dominant frequency, so as to obtain a filtered frequency-domain expression ($FFT_f(g_{AC})$);
      - div) on the basis of the filtered frequency-domain expression, obtaining a sinusoidal time-domain function ($sin_f$) having the dominant frequency;
      - dv) taking into account a selection condition and selecting characteristic times ($t'_n$) of the sinusoidal time-domain function resulting from div), each characteristic time corresponding to a time at which said sinusoidal time-domain function ($sin_f$) meets the selection condition;
      - dvi) on the basis of the characteristic times ($t'_n$) of the sinusoidal time-domain function, selecting characteristic times ($t_n$) of the pulsed component, each characteristic time of the pulsed component ($g_{AC}$) corresponding to a time at which said pulsed component meets the selection condition;
      - dvii) determining the frequency or period of the time-domain variation in the physiological parameter on the basis of the characteristic times ($t_n$) of the pulsed component resulting from dvi).

2. The method of claim 1, wherein:

   - diii) comprises carrying out bandpass filtering on the frequency-domain expression resulting from di), in a passband containing the dominant frequency selected in dii), so as to obtain a filtered frequency-domain expression;
   - div) comprises computing a time-domain expression for the filtered frequency-domain expression ($FFT^{-1}_f(g_{AC})$), the computed time-domain expression corresponding to the sinusoidal function ($sin_f$).

3. The method of any one of the preceding claims, wherein:

   - in dv), the selection condition is reaching an extremum, the extremum either being a maximum, or a minimum, each characteristic time ($t'_n$) being one extremum of the sinusoidal function ($sin_f$);
   - in dvi), each characteristic time $t_n$) is one extremum of the pulsed component ($g_{AC}$).

4. The method of any one of the preceding claims, wherein step dvi) comprises:

   - defining a time interval ($\delta t$) about each characteristic time selected on the basis of the sinusoidal time-domain function, the time interval being shorter than one period of the sinusoidal time-domain function;
- in each time interval, selecting a time $(t_n)$ at which the pulsed component meets the selection condition.

5. The method of any one of claims 1 to 3, wherein:

- step div) implements a dynamic-time-warping algorithm, so as to optimize a match between the sinusoidal function $(sin_f)$ and the pulsed component;
- in dvi), the characteristic times $(t_n)$ of the pulsed component $(g_{AC})$ correspond to the characteristic times $(t'_n)$ of the sinusoidal function $(sin_f)$.

6. The method of any one of the preceding claims, wherein step dvii) comprises estimating a heart rate or period.

7. The method of claim 6, wherein the heart rate or heart period is estimated on the basis of lengths of time separating successive characteristic times $(t_n, t_{n+1})$ of the pulsed component $(g_{AC})$.

8. The method of claim 7, wherein dvii) comprises:

- computing a time-domain variation in the heart rate or period $(HR(t))$;
- determining at least one period (T) of the time-domain variation in the heart rate or period;
- estimating a breathing rate $(RR(t))$ or period on the basis of the period of said time-domain variation.

9. The method of claim 8, wherein step dvii) comprises applying smoothing to the time-domain variation in the heart rate, prior to determining the breathing rate or period.

10. The method of any one of the preceding claims, wherein the selection condition taken into account in dv) and dvi) is reaching an extremum, the method comprising a step of characterizing cardiac or respiratory activity, comprising:

- forming a baseline $(BL)$,, passing through the time-domain measurement function $(g)$ or the pulsed component $(g_{AC})$, at each characteristic time $(t_n)$ resulting from dvi);
- obtaining a characterization function (h), by subtracting the time-domain measurement function or the pulsed component from the baseline, the characterization function comprising lobes, each lobe extending between two successive characteristic times;
- characterizing cardiac or respiratory activity on the basis of a shape or of an area of the lobes of the characterization function.

11. A device for determining a frequency or period of a time-domain variation in a physiological parameter, the physiological parameter varying periodically as a function of time, under the effect of a cardiac activity or respiratory activity of a user, the device comprising:

- a light source (10) arranged to emit light toward the bodily region;
- a photodetector (20), arranged to detect an intensity of light emitted by the light source and having propagated through the bodily region;
- a processing unit (30), configured to implement steps b) to d) of a method of any one of the preceding claims, on the basis of light intensities detected by the photodetector at the various times.

**Fig. 1A**

**Fig. 1B**

**Fig. 1C**

**Fig. 2A**

**Fig. 2B**

**Fig. 3**

**Fig. 4A**

**Fig. 4B**

**Fig. 4C**

**Fig. 4D**

**Fig. 4E**

**Fig. 5A**

**Fig. 5B**

**Fig. 5C**

**Fig. 5D**

Fig. 6A

Fig. 6B

Fig. 6C

Fig. 7A

Fig. 7B

Fig. 7C

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 9778111 B2
- US 20150155015 A

- US 201501555015 A